# EUROPÄISCHE PATENTSCHRIFT

(11) **EP 2 791 126 B1**
(45) Veröffentlichungstag und Bekanntmachung des Hinweises auf die Patenterteilung: **01.03.2017**
(21) Anmeldenummer: 12808306.0
(22) Anmeldetag: 11.12.2012
(51) Int. Cl.: C07D 307/46

(54) **HERSTELLUNG VON 5-HYDROXYMETHYLFURFURAL (HMF) AUS HEXOSELÖSUNGEN IN GEGENWART VON WASSERDAMPF**
PREPARATION OF 5-HYDROXYMETHYLFURFURAL (HMF) FROM HEXOSE SOLUTIONS IN THE PRESENCE OF STEAM
PRODUCTION DE 5-HYDROXYMÉTHYLFURFURAL (HMF) À PARTIR DE SOLUTIONS D'HEXOSE EN PRÉSENCE DE VAPEUR D'EAU

(30) Priorität: 13.12.2011 EP 11193157
(43) Veröffentlichungstag der Anmeldung: 22.10.2014
(73) Patentinhaber: BASF SE, 67056 Ludwigshafen am Rhein (DE)
(72) Erfinder: BACKES, René, 68623 Lamperheim (DE); BLANK, Benoit, 68167 Mannheim (DE); KINDLER, Alois, 67269 Grünstadt (DE); FELDNER, Carmen, 67069 Ludwigshafen (DE)
(74) Vertreter: BASF IP Association
(86) Internationale Anmeldenummer: PCT/EP2012/075055
(87) Internationale Veröffentlichungsnummer: WO 2013/087613

(56) Entgegenhaltungen:
- EP-A1- 2 033 958
- CN-A- 102 399 203
- FR-A1- 2 663 933
- US-A- 2 929 823
- US-A- 3 201 331
- KUSTER B F M: "5-Hydroxymethylfurfural (HMF). A Review Focussing on its manufacture", STARKE - STARCH, WILEY-VCH VERLAG, WEINHEIM, DE, Bd. 42, Nr. 8, 1. Januar 1990 (1990-01-01) , Seiten 314-321, XP002463454, ISSN: 0038-9056, DOI: 10.1002/STAR.19900420808
- JUBEN N CHHEDA ET AL: "Production of 5-hydroxymethylfurfural and furfural by dehydration of biomass-derived mono- and poly-saccharides", GREEN CHEMISTRY, ROYAL SOCIETY OF CHEMISTRY, CAMBRIDGE, GB, Bd. 9, Nr. 4, 1. Januar 2007 (2007-01-01), Seiten 342-350, XP008142504, ISSN: 1463-9262, DOI: 10.1039/B611568C [gefunden am 2007-01-17]
- DANIEL BETHGE: 'Reaktivdestillation eröffnet neue Perspektiven', [Online] 30 April 2010, XP055235758 Gefunden im Internet: <URL:http://chemanager-online.com> [gefunden am 2015-12-11]

## Beschreibung

Die vorliegenden Erfindung betrifft ein Verfahren zur Herstellung von 5-Hydroxymethylfurfural (HMF), welches dadurch gekennzeichnet ist, dass
a) Lösungen (im Nachfolgenden Ausgangs-Lösung genannt), welche
   - eine Hexose und
   - ein organisches Lösemittel mit einem Siedepunkt größer 200°C (bei Normaldruck) enthalten (kurz Hochsieder genannt),
   und Wasserdampf einem Reaktionsgefäß zugeführt werden,
b) in dem Reaktionsgefäß eine Umsetzung der Hexose zu HMF in Gegenwart von Wasserdampf bei gleichzeitiger destillativer Abtrennung des HMF erfolgt und
c) als Destillat eine wässrige, HMF- enthaltende Lösung (im Nachfolgenden Destillat genannt) erhalten wird.

Für chemische Synthesen sind zunehmend Verbindungen von Bedeutung, die aus nachwachsenden Rohstoffen erhalten werden und durch chemische Umsetzungen leicht in technisch anwendbare Verbindungen überführt werden können.

In diesem Zusammenhang ist 5-Hydroxymethylfurfural (HMF) bekannt, welches durch unterschiedliche Verfahren aus Hexosen hergestellt werden kann. Aus HMF ist z.B. leicht 2,5-Furandicarbonsäure erhältlich, welche als Dicarbonsäure zur Herstellung von Polymeren, wie Polyester oder Polyurethane, geeignet ist, und andere Dicarbonsäuren aus nicht nachwachsenden Rohstoffen in technischen Anwendungen ersetzen kann.

HMF wird in der Regel durch sauer katalysierte Dehydratisierung von Hexosen wie Glucose oder Fructose hergestellt.
Als Reaktionsprodukt werden saure Lösungen erhalten, welche neben dem HMF nicht umgesetzte Ausgangsstoffe und/oder Nebenprodukte enthalten. Bei der HMF-Synthese erfolgt in der Regel nur ein Teilumsatz der Ausgangsstoffe, um die Bildung von Nebenprodukten zu vermeiden. Im Allgemeinen enthalten die erhaltenen Lösungen daher nicht umgesetzte Ausgangsstoffe wie Hexosen oder aus Hexosen aufgebaute Oligomere oder Polymere. Bei höheren Umsätzen nimmt die Menge an Nebenprodukten zu.

Die Abtrennung des HMF aus der Reaktionslösung, welche Ausgangsstoffe oder Nebenprodukte der HMF-Synthese enthalten, ist aufwendig und erschwert die Zugänglichkeit von HMF.

So beschreiben Feroz Kabir Kazi et al. in Chem. Eng. J. 169 (2011), Seiten 329-338 die Abtrennung des HMF von der sauren Reaktionslösung durch ein aufwendiges Extraktionsverfahren unter Verwendung eines organischen Lösemittels (Butanol); es wird eine Lösung von HMF in Butanol erhalten.

Aus DE-A 3601281 ist ein chromatographisches Abtrennverfahren bekannt, bei dem zunächst jegliche organische Lösemittel entfernt werden und die wässrige HMF-Lösung mit einer Ionenaustauschersäule aufgetrennt wird. Die gewonnene HMF-Fraktion wird kristallisiert.

Eine weitere Methode, HMF aus der Reaktionslösung abzutrennen, ist die Umwandlung des HMF in eine andere, leichter abtrennbare Verbindung, gegebenenfalls gefolgt von einer Rückumwandlung in HMF nach erfolgter Abtrennung. So wird HMF nach Mark Mascal und Edward B.Nikitin in 2008 Angew. Chemie Vol. 47, Seiten 7924-7926 in das stabilere 5-Chlormethylfurfural umgewandelt und anschließend wieder in HMF oder dessen Derivate überführt. Alternativ werden nach EP-A 1834950 die Ether bzw. nach EP-A 1834951 die Ester des HMF hergestellt, welche sich nach erfolgter Abtrennung direkt für weitere Synthesen eignen.

Haru Kawamoto, Shinya Saito et al. beschreiben in J. Wood Sci. (2007), 53, Seiten 127 - 133 die Pyrolyse von Cellulose unter Bildung von Levoglucosenon, Furfural und/oder HMF unter verschiedenen Bedingungen, auch bei Zufuhr von Wasserdampf.

EP-A1-2033958 erwähnt die Möglichkeit einer katalytischen Reaktivdestillation für die Herstellung von HMF.

HMF sollte für weitere Synthesen in möglichst reiner Form vorliegen. Für weitere Synthesen sind insbesondere wässrige Lösung von HMF geeignet, welche Nebenprodukte oder restliche Ausgangsstoffe nicht oder allenfalls in sehr geringen Mengen enthält. Bisher bekannte Verfahren, HMF oder dessen wässrige Lösungen mit ausreichender Reinheit herzustellen, sind äußerst aufwendig.

Aufgabe der vorliegenden Erfindung war daher ein Verfahren, mit dem HMF in möglichst einfacher und effektiver Weise hergestellt werden kann, HMF gleichzeitig in möglichst reiner Form erhalten wird und HMF daher von umgesetzten Ausgangstoffen oder Nebenprodukten der Synthese unmittelbar möglichst vollständig abgetrennt wird.

Demgemäß wurde das eingangs definierte Verfahren gefunden.

Bei dem erfindungsgemäßen Verfahren wird HMF in Gegenwart von Wasserdampf hergestellt und unmittelbar von Nebenprodukten und nicht umgesetzten Ausgangsstoffen der HMF-Synthese abgetrennt.

### Zu Verfahrensschritt a)

Im Verfahrensschritt a) werden Lösungen (im Nachfolgenden Ausgangs-Lösung genannt), welche
- eine Hexose und
- ein organisches Lösemittel mit einem Siedepunkt größer 200°C (bei Normaldruck) enthalten (kurz Hochsieder genannt),
und Wasserdampf einem Reaktionsgefäß zugeführt.

Bei der Hexose handelt es sich vorzugsweise um Fructose, Glucose oder Gemische von Fructose und Glucose. Besonders bevorzugt handelt es sich um Fructose oder Gemische von Fructose mit Glucose.

Die Ausgangs-Lösung kann auch Nebenprodukte oder Ausgangsprodukte aus der Herstellung der Hexose enthalten. Zum Beispiel können Hexosen durch Abbau von Polymeren wie Cellulose oder Stärke gewonnen werden. Daher kann die Ausgangs-Lösung noch Restmengen an derartigen Polymeren oder deren oligomere Abbauprodukte enthalten.

Vorzugsweise enthält die Ausgangs-Lösung 1 bis 40 Gew. % Hexose, besonders bevorzugt 5 bis 30 Gew. % Hexose, bezogen auf das Gesamtgewicht der Ausgangs-Lösung.

Vorzugsweise enthält die Ausgangslösung weniger als 10 Gew. %, insbesondere weniger als 5 Gew. % und besonders bevorzugt weniger als 1 Gew. % an Nebenprodukten oder Ausgangsstoffen aus der Herstellung von Hexosen. Insbesondere ist die Ausgangs-Lösung im Wesentlichen frei von Nebenprodukten und Ausgangsstoffen aus der Herstellung von Hexosen.

Die Ausgangs-Lösung enthält weiterhin ein organisches Lösemittel mit einem Siedepunkt größer 200°C (bei Normaldruck), insbesondere größer 250°C (im Nachfolgenden kurz Hochsieder genannt).

Als Hochsieder in Betracht kommen hydrophile Lösemittel; es kann sich um protische, hydrophile organische Lösemittel, z. B. Alkohole, oder aprotische hydrophile Lösemittel, z. B. Ether oder Ketone, wie Dimethylsulfoxid, handeln.

Im Rahmen dieser Erfindung bevorzugte Hochsieder sind Polyether. Die Polyether haben vorzugsweise einen Schmelzpunkt kleiner 60°C, insbesondere kleiner 30°C (bei Normaldruck, 1 bar); besonders bevorzugte Polyether sind bei 20°C (Normaldruck) flüssig.

Die Polyether enthalten mindestens zwei Ethergruppen. Vorzugsweise enthalten die Polyether mindestens 3, insbesondere mindestens 4, besonders bevorzugt mindestens 6 Ethergruppen. Im Allgemeinen enthalten sie nicht mehr als 40, insbesondere nicht mehr als 30 Ethergruppen, besonders bevorzugt nicht mehr als 20 Ethergruppen.

In einer besonderen Ausführungsform enthalten die Polyether keine Heteroatome außer Sauerstoff in Form von Ethergruppen und gegebenenfalls Hydroxylgruppen.

Insbesondere handelt es sich um aliphatische Polyether, besonders bevorzugte Polyether sind Polyalkylenglycole, wobei die endständigen Hydroxylgruppen mit Alkylgruppen, insbesondere C1- bis C4-Alkylgruppen verethert sein können.

Bei den Alkylengruppen der Polyalkylenglycole kann es sich z.B. um C2- bis C10-, insbesondere um C2- bis C4-Alkylengruppen, wie Ethylen-, Propylen- oder Butylengruppen handeln. Die Polalkylenglycole können auch verschiedene Alkylengruppen enthalten, z.B. in Form von Blöcken.

Ganz besonders bevorzugt sind daher Poly-C2-bis C4-alkylenglycole, insbesondere Polyethylenglycol, deren endständige Hydroxylgruppen gegebenenfalls mit Alkylgruppen verethert sein können; die Anzahl der wiederkehrenden Alkylenethergruppen entspricht der vorstehenden Anzahl der Ethergruppen, insbesondere ist die Anzahl der wiederkehrenden Alkylenethergruppen 4 bis 30, besonders bevorzugt 6 bis 20. Die endständigen Hydroxylgruppen der Polyalkylenglycole können mit Alkylgruppen, insbesondere C1-bis C4-Alkylgruppen, verethert sein.

Die Ausgangs-Lösung kann den vorstehenden Hochsieder als einziges Lösemittel enthalten. In diesem Fall ist die Hexose in dem Hochsieder gelöst.

In einer bevorzugten Ausführungsform enthält die Ausgangs-Lösung neben dem Hochsieder mindestens ein weiteres Lösemittel. Bei dem weiteren Lösemittel kann es sich insbesondere um Wasser oder hydrophile organische Lösemittel mit einem Siedepunkt kleiner 200°C (Niedrigsieder), in denen die verwendete Hexose vorzugsweise löslich sein sollte, oder Gemischen von Wasser mit Niedrigsiedern handeln.

Besonders bevorzugt handelt es sich bei dem weiteren Lösemittel um Wasser. Die Ausgangs-Lösung ist daher besonders bevorzugt eine wässrige Lösung.

In einer besonderen Ausführungsform enthält die Ausgangslösung als Lösemittel ausschließlich Wasser und den Hochsieder.

Die Ausgangs-Lösung enthält den Hochsieder, insbesondere den Polyether, vorzugsweise in Mengen von 5 bis 90 Gew. %, insbesondere von 30 bis 80 Gew. %, besonders bevorzugt von 50 bis 70 Gew. %, bezogen auf das Gesamtgewicht der Ausgangs-Lösung

Vorzugsweise ist der Gehalt der Ausgangs-Lösung an Niedrigsiedern kleiner 50 Gew. %, insbesondere kleiner 30 Gew. % und besonders bevorzugt kleiner 20 Gew.%.

Die Ausgangs-Lösung enthält weiterhin vorzugsweise eine Säure. Säuren katalysieren die Umsetzung von Hexose zu HMF. Als Säuren in Betracht kommen heterogene Säuren, welche in der Ausgangs-Lösung dispergiert sind oder homogene Säuren, welche in der Ausgangs-Lösung gelöst sind.

Vorzugsweise enthält die Ausgangs-Lösung eine homogene Säure. Als homogene Säuren kommen beliebige anorganische oder organische Säuren in Betracht. Exemplarisch genannt seien para-Toluolsulfonsäure, Methansulfonsäure (MeOSO₃H), Oxalsäure, Schwefelsäure, Salzsäure oder Phosphorsäure. Die Ausgangs-Lösung enthält die Säure vorzugsweise in Mengen von 0,1 bis 10 mol% (bezogen auf die Hexose), besonders bevorzugt von 0,1 bis 5 mol%.

Bevorzugte Ausgangs-Lösungen enthalten z. B.
1 bis 40 Gew. % Hexose
5 bis 90 Gew. % Hochsieder, vorzugsweise Polyether
1 bis 50 Gew. % Wasser
0,1 bis 10 mol % Säure (bezogen auf die Hexose)
0 bis 10 Gew. % sonstige Bestandteile, z. B. Nebenprodukte aus der Synthese der Hexose,
bezogen auf das Gesamtgewicht der Lösung.

Besonders bevorzugte Ausgangs-Lösungen enthalten z. B.
5 bis 30 Gew. % Hexose
30 bis 80 Gew. % Hochsieder, vorzugsweise Polyether
10 bis 50 Gew. % Wasser
0,1 bis 5 mol % Säure (bezogen auf die Hexose)
0 bis 5 Gew. % sonstige Bestandteile, z. B. Nebenprodukte aus der Synthese der Hexose,
bezogen auf das Gesamtgewicht der Lösung.

Die vorstehend beschriebene Ausgangs-Lösung und Wasserdampf werden einem Reaktionsgefäß zugeführt.

### Zu Verfahrensschritt b)

In Verfahrensschritt b) erfolgt die Umsetzung der Ausgangs-Lösung zu HMF in Verbindung mit einer für sich genommen bekannten Wasserdampfdestillation. Dazu wird die Ausgangs-Lösung mit dem Wasserdampf im Reaktionsgefäß in Kontakt gebracht.

Die Behandlung der Ausgangs-Lösung mit Wasserdampf erfolgt vorzugsweise bei vermindertem Druck, in Betracht kommt insbesondere ein Druck von 1 bis 300 mbar. Insbesondere beträgt der Druck im Reaktionsgefäß 1 bis 100 mbar, besonders bevorzugt 1 bis 50 mbar und in einer ganz besonders bevorzugten Ausführungsform 1 bis 40 bzw. 1 bis 35 mbar.

Die Behandlung der Ausgangs-Lösung mit Wasserdampf erfolgt vorzugsweise bei einer Temperatur der Ausgangs-Lösung von 100 bis 200°C, besonders bevorzugt von 120 bis 180°C und besonders bevorzugt von 140 bis 180°C und ganz besonders bevorzugt 150 bis 180°C.

Vorzugsweise wird das erfindungsgemäße Verfahren kontinuierlich betrieben.

Dazu wird die Ausgangs-Lösung und der Wasserdampf dem Reaktionsgefäß kontinuierlich zugeführt und das erhaltene Produkt, bzw. Destillat, kontinuierlich abgeführt.

Die Volumenströme hängen von der Größe und Trennleistung des gewählten Reaktionsgefäßes ab.

In einer bevorzugten Ausführungsform liegt das Verhältnis des zugeführten Volumen an Wasserdampf zum Volumen der zugeführten Ausgangs-Lösung in einem Bereich von 0,5 bis 2 Volumeneinheiten Wasserdampf auf 1 Volumeneinheit Ausgangs-Lösung, besonders bevorzugt im Bereich von 0,8 bis 1,5 Volumeneinheiten Wasserdampf auf 1 Volumeneinheit Ausgangs-Lösung und insbesondere 0,8 bis 1,2 Volumeneinheiten Wasserdampf auf 1 Volumeneinheit Ausgangs-Lösung.

Als Reaktionsgefäß eigenen sich übliche Verdampfer, welche für die Zufuhr von Ausgangs-Lösung und Wasserdampf und insbesondere für vorstehend beschriebene kontinuierliche Verfahrensweise eingerichtet sind.

Bevorzugte Verdampfer sind Dünnschichtverdampfer. Bei denen die Ausgangs-Lösung im Verdampfer als Flüssigkeitsfilm vorliegt.

Besonders bevorzugt sind vertikale Dünnschichtverdampfer; derartige vertikale Dünnschichtverdampfer sind unter Gerätebezeichnungen wie "Luwa" oder insbesondere "Sambay" bekannt.

Bei den bevorzugten vertikalen Dünnschichtverdampfern handelt es sich letztlich um ein senkrechtes Rohr mit innenliegenden Vorrichtungen zur Verteilung und Durchmischung der Ausgang-Lösung und außenliegenden Vorrichtungen zur Beheizung der Rohrwand.

Die Ausgangs-Lösung wird vorzugsweise im oberen Teil des Dünnschichtverdampfers zugeführt und als Film auf die beheizte Rohrwand verteilt. Wasserdampf kann dem Verdampfer, vorzugsweise dem Dünnschichtverdampfer, zusammen mit der Ausgangs-Lösung oder an einer beliebigen anderen Stelle des Verdampfers zugeführt werden. Die Ausgangs-Lösung und der Wasserdampf können in dem Verdampfer in gleicher Richtung (Gleichstrom) oder entgegengesetzt (Gegenstrom) geführt werden.

Vorzugsweise wird der Wasserdampf im Gegenstrom zur Ausgangs-Lösung geführt. Dazu werden die Ausgangs-Lösung insbesondere im oberen Teil des Verdampfers und der Wasserdampf im unteren Teil des Verdampfers zugeführt.

Der Wasserdampf und die flüchtigen Bestandteile der Ausgangs-Lösung werden vorzugsweise über einen Abscheider am Kopf des Verdampfers ausgeführt und kondensiert (Destillat).

Die nichtflüchtigen Bestandteile durchlaufen den Verdampfer und werden als flüssiges Sumpfprodukt abgetrennt.

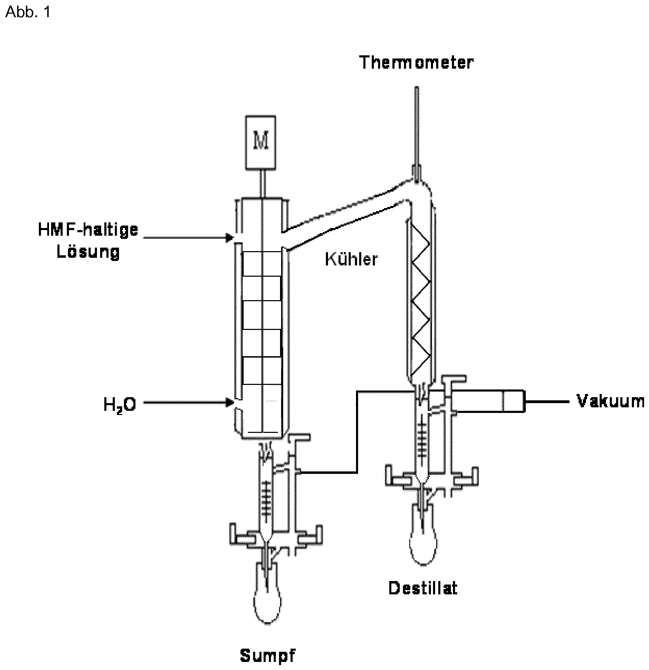

Abbildung 1 zeigt eine entsprechende Apparatur aus Dünnschichtverdampfer (Sambay) und Vorrichtung zur Kondensation.

Die Reaktion in Verfahrensschritt b) kann je nach Wunsch so durchgeführt werden, dass nur ein Teilumsatz der Hexose zum HMF oder ein vollständiger Umsatz der Hexose zu HMF erfolgt. Bei einem Teilumsatz kann nicht umgesetzte Hexose erneut umgesetzt werden, bei einem vollständigen Umsatz kann es vermehrt zur Bildung von Nebenprodukten, insbesondere sogenannten Huminen, das sind Oligomere des HMF, kommen.

Vorzugsweise werden mindestens 40 Gew. %, insbesondere mindestens 60 Gew.% und in einer besonderen Ausführungsform mindestens 80 Gew. % der Hexose umgesetzt.

### Zu Verfahrensschritt c)

Als Destillat wird eine wässrige, HMF- enthaltende Lösung erhalten. Das Destillat enthält das bei der Umsetzung entstandene HMF und Wasser aus der Wasserdampfdestillation.

Das Destillat enthält insbesondere mehr als 60%, insbesondere mehr als 80% des bei der Umsetzung insgesamt erhaltenen HMF.

Darüber hinaus kann das Destillat auch Hochsieder enthalten. Im Falle der Verwendung von Polyether als Hochsieder, enthält das Destillat keinen oder nur geringe Mengen an Hochsieder; der Gehalt an Polyether im Destillat ist dann insbesondere kleiner 5 Gew. %, vorzugsweise kleiner 2 Gew. % und besonders bevorzugt kleiner 1 bzw. kleiner 0,5 Gew. %, bezogen auf das Gesamtgewicht des Destillats.

Nebenprodukte, die bei der Umsetzung der Hexose zu HMF entstehen sind insbesondere Humine (Oligomere des HMF). Die Humine fallen beim erfindungsgemäßen Verfahren im Wesentlichen nicht im Destillat sondern im Sumpf (siehe Abbildung 1) an.

Das Destillat enthält daher keine oder nur sehr geringe Mengen an Huminen; der Gehalt an Huminen im Destillat ist im Allgemeinen kleiner 2, insbesondere kleiner 0,5 und besonders bevorzugt kleiner 0,1 Gew. %. Das Destillat ist klar und hat eine leichte Gelbfärbung.

Weiterhin enthält das Destillat keine oder nur geringe Mengen an nicht umgesetzter Hexose; nicht umgesetzte Hexose befindet sich überwiegend im Sumpf.

Der Gehalt an nicht umgesetzter Hexose im Destillat ist im Allgemeinen kleiner 5 Gew. %, insbesondere kleiner 2 Gew. % und besonders bevorzugt kleiner 1 Gew. %.

Es ist ein Vorteil des erfindungsgemäßen Verfahrens, dass Nebenprodukte der HMF Synthese, Polyether als Hochsieder und nicht umgesetzte Hexose im Wesentlichen im Sumpf anfallen.

HMF wird beim erfindungsgemäßen Herstellungsverfahren direkt als Destillat mit hoher Reinheit erhalten. Das erfindungsgemäße Verfahren ist daher eine einfaches und effektives Verfahren zur Herstellung von HMF und gleichzeitigen Abtrennung von HMF von Nebenprodukten und nicht umgesetzten Ausgangsstoffen.

Das Destillat eignet sich für chemische Synthesen, bei denen HMF als Ausgangsstoff eingesetzt wird. Insbesondere eignet sich das Destillat für chemische Synthesen bei denen der Ausgangsstoff HMF in hoher Reinheit gewünscht oder benötigt wird. Exemplarisch sei hier die Verwendung der Produkt-Lösung zur Herstellung von 2,5-Furandicarbonsäure oder von 2,5-Bis-(hydroxymethyl)furan genannt.

### Beispiele

### Beispiel 1:

in-situ Dehydratisierung von Fructose und Isolation von HMF mittels Wasserdampfdestillation

### Ausgangs-Lösung

Die Ausgangs-Lösungen wurden erhalten durch Mischen von Reinsubstanzen.
Die Ausgangs-Lösungen enthielten Fructose, Hochsieder, Säure und Wasser (siehe Tabelle).

Als Hochsieder wurden verwendet:
DMSO: Dimethylsulfoxid
PEG-600: ein Polyethylenglycol mit einem Molekulargewicht von 600
Tetraglyme: Tetraethylenglycoldimethylether

Als Säuren wurden verwendet:
H2SO4: Schwefelsäure
p-TSA: para-Toluolsulfonsäure
MSA: Methansulfonsäure
Oxalsäure

### Durchführung der Wasserdampfdestillation

Die Wasserdampfdestillation wurde in der Apparatur gemäß Figur 1 durchgeführt. Die Apparatur besteht aus einem Glassambay, der in Gegenstromfahrweise betrieben wird.

Die Ausgangs-Lösung wurde am Kopf zugeführt, der Wasserdampf im unteren Drittel.

Zusammensetzung der Ausgangs-Lösung für verschiedene Hochsieder sowie die gewählten Temperaturen und Drücke sind in der Tabelle aufgeführt.

Die angegebene Temperatur ist diejenige des Heizmediums an der Rohraußenwand, welche in guter Näherung derjenigen des flüssigen Film der Ausgangs-Lösung an der Rohrinnenwand entspricht.

Die Versuche wurden kontinuierlich durchgeführt, nach jeder neuen Temperatur- und Druckeinstellung wurde gewartet bis ein stationärer Zustand erreicht war.

Die Zusammensetzung wurde mittels HPLC bestimmt.

Die angegebenen Umsätze an Fructose ergeben sich aus den Restmengen an Fructose im Sumpf und Destillat; Fructose wurde umgesetzt zu HMF und zu Nebenprodukten (Huminen). Die angegebenen katalytischen Mengen an Säure sind bezogen auf Fructose. Die Ausbeute HMF ist der prozentuale Anteil des HMF im Destillat bzw. im Sumpf, bezogen auf den Fructose-Gehalt in der Ausgangs-Lösung.

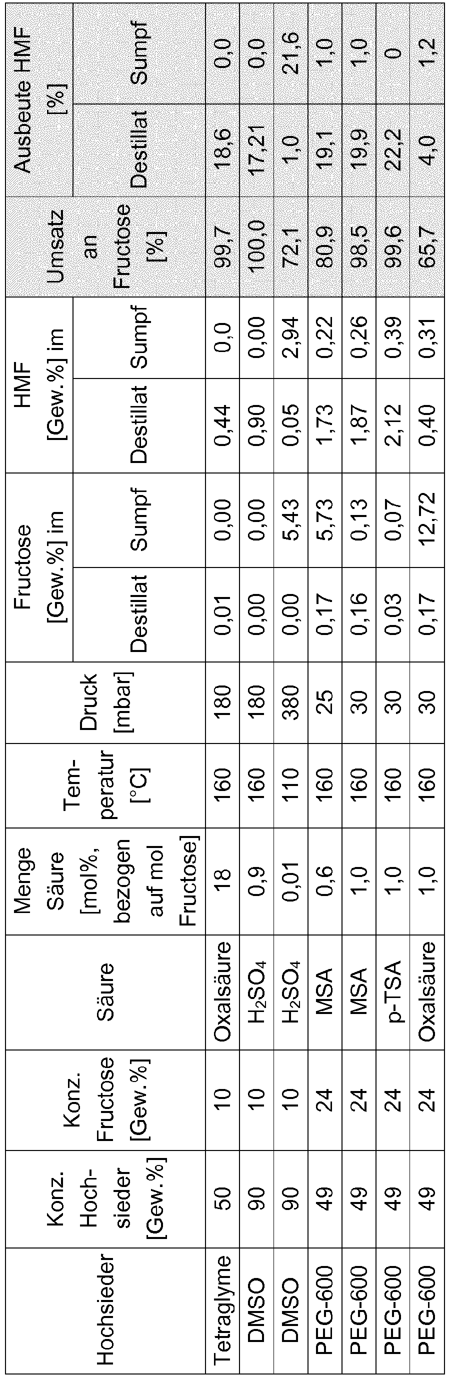

## Patentansprüche

1. Verfahren zur Herstellung von 5-Hydroxymethylfurfural (HMF), **dadurch gekennzeichnet, dass**
a) Lösungen (im Nachfolgenden Ausgangs-Lösung genannt), welche
- eine Hexose und
- ein organisches Lösemittel mit einem Siedepunkt größer 200°C (bei Normaldruck) enthalten (kurz Hochsieder genannt),
und Wasserdampf einem Reaktionsgefäß zugeführt werden,
b) in dem Reaktionsgefäß eine Umsetzung der Hexose zu HMF in Gegenwart von Wasserdampf bei gleichzeitiger destillativer Abtrennung des HMF erfolgt und
c) als Destillat eine wässrige, HMF- enthaltende Lösung (im Nachfolgenden Destillat genannt) erhalten wird.

2. Verfahren gemäß Anspruch 1, **dadurch gekennzeichnet, dass** das Verhältnis des zugeführten Volumen an Wasserdampf zum Volumen der zugeführten Ausgangs-Lösung in einem Bereich von 0,5 bis 2 Volumeneinheiten Wasserdampf auf 1 Volumeneinheit Ausgangs-Lösung liegt.

3. Verfahren gemäß Anspruch 1 oder 2, **dadurch gekennzeichnet, dass** die Ausgangs-Lösung weiterhin eine Säure enthält.

4. Verfahren gemäß Anspruch 1, 2 oder 3, **dadurch gekennzeichnet, dass** es sich bei der Hexose um Fructose, Glucose oder Gemische von Fructose und Glucose handelt.

5. Verfahren gemäß einem der vorigen Ansprüche, **dadurch gekennzeichnet, dass** es sich bei dem Hochsieder um einen Polyether handelt:

6. Verfahren gemäß einem der vorigen Ansprüche, **dadurch gekennzeichnet, dass** es sich bei dem Polyether um ein Poly- C2-bis C4-alkylen-glycol, dessen endständige Hydroxylgruppen gegebenenfalls mit C1-C4 Alkylgruppen verethert sind, handelt:

7. Verfahren gemäß einem der vorigen Ansprüche, **dadurch gekennzeichnet, dass** die Ausgangs-Lösung den Hochsieder in Mengen von 5 bis 90 Gew. % enthält.

8. Verfahren gemäß einem der vorigen Ansprüche, **dadurch gekennzeichnet, dass** es sich bei der Ausgangs-Lösung um eine wässrige Lösung handelt.

9. Verfahren gemäß einem der vorigen Ansprüche, **dadurch gekennzeichnet, dass** die Umsetzung zu HMF in Gegenwart einer in der Ausgangs-Lösung löslichen Säure erfolgt.

10. Verfahren gemäß einem der vorigen Ansprüche, **dadurch gekennzeichnet, dass** die Umsetzung zu HMF bei 100°C bis 200°C erfolgt.

11. Verfahren gemäß einem der vorigen Ansprüche, **dadurch gekennzeichnet, dass** die Umsetzung zu HMF bei einem Druck von 1 bis 300 mbar erfolgt.

12. Verfahren gemäß einem der vorigen Ansprüche, **dadurch gekennzeichnet, dass** das Verfahren kontinuierlich durchgeführt wird, wobei die Ausgangslösung und Wasserdampf dem Verdampfer kontinuierlich zugeführt werden und die Produkt-Lösung kontinuierlich abgeführt wird.

13. Verfahren gemäß einem der vorigen Ansprüche, **dadurch gekennzeichnet, dass** es sich bei dem Reaktionsgefäß um einen Dünnschichtverdampfer handelt.

14. Verfahren gemäß einem der vorigen Ansprüche, **dadurch gekennzeichnet, dass** der Wasserdampf im Gegenstrom zur Ausgangs-Lösung zugeführt wird.

15. Verfahren gemäß einem der vorigen Ansprüche, **dadurch gekennzeichnet, dass** sich mehr als 60% des erhaltenen HMF im Destillat befinden.

16. Verfahren gemäß einem der vorigen Ansprüche, **dadurch gekennzeichnet, dass** mindestens 40 Gew. % der Hexose umgesetzt werden.

17. Verfahren gemäß einem der vorigen Ansprüche, **dadurch gekennzeichnet, dass** das Destillat zur Herstellung von 2,5-Furandicarbonsäure oder 2,5-Bis(hydroxymethyl)furan verwendet wird.

## Claims

1. A method for producing 5-hydroxymethylfurfural (HMF), wherein
a) solutions (hereinbelow called starting solution) which comprise
- a hexose and
- an organic solvent with a boiling point greater than 200°C (at standard pressure) (for short called high-boiling component), and steam are fed to a reaction vessel,
b) in the reaction vessel, a conversion of the hexose to HMF takes place in the presence of steam with the simultaneous distillative removal of the HMF and
c) as distillate, an aqueous, HMF-comprising solution (hereinbelow called distillate) is obtained.

2. The method according to claim 1, wherein the ratio of the fed volume of steam to the volume of the fed starting solution is in a range from 0.5 to 2 volume units of steam to 1 volume unit of starting solution.

3. The method according to claim 1 or 2, wherein the starting solution further comprises an acid.

4. The method according to claim 1, 2 or 3, wherein the hexose is fructose, glucose or mixtures of fructose and glucose.

5. The method according to any one of the preceding claims, wherein the high-boiling component is a polyether.

6. The method according to any one of the preceding claims, wherein the polyether is a poly-C2- to C4-alkylene glycol, the terminal hydroxyl groups of which are optionally etherified with C1-C4 alkyl groups.

7. The method according to any one of the preceding claims, wherein the starting solution comprises the high-boiling component in amounts of from 5 to 90% by weight.

8. The method according to any one of the preceding claims, wherein the starting solution is an aqueous solution.

9. The method according to any one of the preceding claims, wherein the reaction to give HMF takes place in the presence of an acid which is soluble in the starting solution.

10. The method according to any one of the preceding claims, wherein the reaction to give HMF takes place at 100°C to 200°C.

11. The method according to any one of the preceding claims, wherein the reaction to give HMF takes place at a pressure of from 1 to 300 mbar.

12. The method according to any one of the preceding claims, wherein the method is carried out continuously, where the starting solution and steam are fed to the evaporator continuously and the product solution is drawn off continuously.

13. The method according to any one of the preceding claims, wherein the reaction vessel is a thin-film evaporator.

14. The method according to any one of the preceding claims, wherein the steam is fed countercurrently to the starting solution.

15. The method according to any one of the preceding claims, wherein more than 60% of the HMF obtained are in the distillate.

16. The method according to any one of the preceding claims, wherein at least 40% by weight of the hexose is reacted.

17. The method according to any one of the preceding claims, wherein the distillate is used for producing 2,5-furandicarboxylic acid or 2,5-bis(hydroxymethyl)furan.

## Revendications

1. Procédé de fabrication de 5-hydroxyméthylfurfural (HMF), **caractérisé en ce que**
a) des solutions (par la suite nommées solution initiale), qui contiennent
- un hexose et
- un solvant organique ayant un point d'ébullition supérieur à 200 °C (à pression normale) (nommé composé de point d'ébullition élevé en abrégé),
et de la vapeur d'eau sont introduites dans un récipient de réaction,
b) une transformation de l'hexose en HMF en présence de vapeur d'eau a lieu dans le récipient de réaction avec séparation par distillation simultanée de l'HMF,
et
c) une solution aqueuse contenant l'HMF (par la suite nommée distillat) est obtenue en tant que distillat.

2. Procédé selon la revendication 1, **caractérisé en ce que** le rapport entre le volume introduit de vapeur d'eau et le volume de solution initiale introduite se situe dans une plage allant de 0,5 à 2 unités volumiques de vapeur d'eau sur 1 unité volumique de solution initiale.

3. Procédé selon la revendication 1 ou 2, **caractérisé en ce que** la solution initiale contient en outre un acide.

4. Procédé selon la revendication 1, 2 ou 3, **caractérisé en ce que** l'hexose est le fructose, le glucose ou des mélanges de fructose et de glucose.

5. Procédé selon l'une quelconque des revendications précédentes, **caractérisé en ce que** le composé de point d'ébullition élevé est un polyéther.

6. Procédé selon l'une quelconque des revendications précédentes, **caractérisé en ce que** le polyéther est un poly-alkylène glycol en C2 à C4, dont les groupes hydroxyle terminaux sont éventuellement éthérifiés avec des groupes alkyle en C1-C4.

7. Procédé selon l'une quelconque des revendications précédentes, **caractérisé en ce que** la solution initiale contient le composé de point d'ébullition élevé en quantités de 5 à 90 % en poids.

8. Procédé selon l'une quelconque des revendications précédentes, **caractérisé en ce que** la solution initiale est une solution aqueuse.

9. Procédé selon l'une quelconque des revendications précédentes, **caractérisé en ce que** la transformation en HMF a lieu en présence d'un acide soluble dans la solution initiale.

10. Procédé selon l'une quelconque des revendications précédentes, **caractérisé en ce que** la transformation en HMF a lieu à 100 °C à 200 °C.

11. Procédé selon l'une quelconque des revendications précédentes, **caractérisé en ce que** la transformation en HMF a lieu à une pression de 1 à 300 mbar.

12. Procédé selon l'une quelconque des revendications précédentes, **caractérisé en ce que** le procédé est réalisé en continu, la solution initiale et la vapeur d'eau étant introduites en continu dans l'évaporateur et la solution de produit étant déchargée en continu.

13. Procédé selon l'une quelconque des revendications précédentes, **caractérisé en ce que** le récipient de réaction est un évaporateur à couches minces.

14. Procédé selon l'une quelconque des revendications précédentes, **caractérisé en ce que** la vapeur d'eau est introduite à contre-courant de la solution initiale.

15. Procédé selon l'une quelconque des revendications précédentes, **caractérisé en ce que** plus de 60 % de l'HMF obtenu se trouve dans le distillat.

16. Procédé selon l'une quelconque des revendications précédentes, **caractérisé en ce qu'**au moins 40 % en poids de l'hexose est transformé.

17. Procédé selon l'une quelconque des revendications précédentes, **caractérisé en ce que** le distillat est utilisé pour la fabrication d'acide 2,5-furanedicarboxylique ou de 2,5-bis(hydroxyméthyl)furane.
